**Europäisches Patentamt**

(19) **European Patent Office**

**Office européen des brevets**

(11) Publication number : **0 370 036 B1**

# (12) EUROPEAN PATENT SPECIFICATION

(45) Date of publication of patent specification :
03.06.92 Bulletin 92/23

(51) Int. Cl.[5] : **C12N 9/64,** C12N 15/57, C12P 21/00

(21) Application number : **88905735.2**

(22) Date of filing : **24.06.88**

(86) International application number :
**PCT/DK88/00103**

(87) International publication number :
**WO 88/10295 29.12.88 Gazette 88/28**

(54) **MODIFIED FACTOR VII/VIIa.**

(30) Priority : **25.06.87 DK 3235/87**

(43) Date of publication of application :
**30.05.90 Bulletin 90/22**

(45) Publication of the grant of the patent :
**03.06.92 Bulletin 92/23**

(84) Designated Contracting States :
**AT BE CH DE FR GB IT LI LU NL SE**

(56) References cited :
EP-A- 022 421
EP-A- 0 201 153
EP-A- 0 233 013
WO-A-86/01538
Chemical abstractsm vol. 106 (1987), abstract 106:46313q, Biochemistry (Tate K), 1987, 26(2) 338-43
Chemical abstracts, vol. 103 (1985), abstract 103:83655m, Proc. Nat. Acad. Sci. USA 1985 (Horwich A.L.), 82(15), 4930-3
J. Clin. Invest. Vol. 76, p. 937-946, published September 1985 (George J. Broze, JR et al) "Monoclonal anti-human factor VIIantobidies".

(73) Proprietor : **NOVO NORDISK A/S**
**Novo Allé**
**DK-2880 Bagsvaerd (DK)**
Proprietor : **ZYMOGENETICS, INC.**
**4225 Roosevelt Way N.E.**
**Seattle, WA 98108 (US)**

(72) Inventor : **NICOLAISEN, Else, Marie**
**Mariendalsvej 23A, lejl. 502**
**DK-2000 Frederiksberg (DK)**
Inventor : **BJORN, Soren, Erik**
**Marie Grubbes Allé 47**
**DK-2800 Lyngby (DK)**
Inventor : **WIBERG,Finn**
**Vinkelvej 16**
**3520 Farum (DK)**
Inventor : **WOODBURY, Richard**
**15464 10th Avenue NE**
**Seattle, WA 98155 (US)**

(74) Representative : **Brown, John David**
**FORRESTER & BOEHMERT**
**Franz-Joseph-Strasse 38**
**W-8000 München 40 (DE)**

## Description

TECHNICAL FIELD

The present invention is related to modified factor VII/VIIa's, DNA sequences coding for such modified factors and a process for their production.

BACKGROUND ART

Factor VIIa is a serine protease that participates in blood coagulation by activating factor X and/or factor IX. Factor VIIa is produced from its precursor, factor VII, which is synthesized in the liver and secreted into the blood where it circulates as a single-chain glycoprotein (Mw = 50,000). Factor VII can in vitro be converted into the two-chain form factor VIIa by factor Xa, factor XIIa, factor IXa or thrombin. In the presence of tissue factor and calcium ions, factor VIIa, in vivo is believed to convert factor X to factor Xa by limited proteolysis. The latter enzyme in turn converts prothrombin to thrombin in the presence of factor Va, calcium ions, and phospholipid. Factor VIIa will also convert factor IX to factor IXa in the presence of tissue factor and calcium.

Factor VII can be purified from plasma and activated into factor VIIa by the methods described by Broze and Majerus, J. Biol.Chem. 255 (4): 1242 - 1247, 1980 and Hedner and Kisiel, J.Clin.Invest. 71: 1836 - 1841, 1983.

Factor VIIa may also be produced by recombinant DNA-technology by culturing in an appropriate medium mammalian cells transfected with a DNA-sequence encoding factor VII, isolating the protein produced and activating said protein to factor VIIa (vide European patent application No. 86302855.1).

The cDNA coding for human factor VII has been characterized (Hagen et al., Proc.Natl.Acad.Sci. USA, 83: 2412 - 2416, 1986). The amino acid sequence deduced from the cDNAs indicates that factor VII is synthesized with a prepro-leader sequence of 60 or 38 amino acids. The mature factor VII that circulates in plasma is composed of 406 amino acid residues. The amino acid sequence analysis of the activated protein and the amino acid sequence deduced from the cDNAs indicate that factor VII is converted to factor VIIa by the cleavage of a single peptide bond between arginine (152) and isoleucine (153). This results in the formation of a two-chained molecule consisting of a light chain (152 amino acid residues) and a heavy chain (254 amino acid residues) that are held together by one disulphide bond. The light chain contains a $\gamma$-carboxyglutamic acid (Gla) domain and two potential epidermal growth factor domains, while the heavy chain contains the serine protease portion of the molecule.

Factor VIIa may be used in treating patients who have developed inhibitors to factor VIII (Hedner, U. and Kisiel, W, J.Clin.Invest., 71: 1836 - 1841, 1983) and for the treatment of patients suffering from bleeding disorders such as platelet disorders including thrombocytopenia, von Willebrand's disease and others typically present in association with severe tissue damages (European patent application No. 86309197.1).

According to observations of the inventors hereof factor VIIa has been found to be a protein susceptible to proteolytic cleavage giving rise to a number of degradation products without clotting activity. The proteolytic cleavage may occur at different steps of the recovery procedure and also during storage. Degradation products have been observed both for factor VIIa derived from plasma as well as for factor VIIa produced by recombinant DNA-technology. The degradation may occur before factor VII has been activated into factor VIIa, i.e. during production and isolation of factor VII, during the activating step itself or during isolation, purification and/or storage of the activated product.

As the degradation products are inactive molecules their occurrence in the factor VIIa preparation will lead to a lower specific activity of the final preparation. Furthermore, the amount and nature of the degradation products may vary from one production batch to another giving rise to preparations with a variable content of biologically active factor VIIa.

Factor VIIa preparations containing inactive degradation products will as mentioned have a less specific activity as compared to preparations in which all or a major part of the protein material is active. Accordingly, higher and more frequent doses are necessary to obtain and sustain a therapeutic or prophylactic effect as compared to a preparation with higher specific·activity.

Variable amounts of inactive degradation products and as a consequence variable content of biologically active factor VIIa will furthermore make calculation of appropriate doses troublesome and difficult, if not in some circumstances impossible.

Finally, a content of non-physiological degradation products in the final preparation may trigger the immune system of the patient. Readministration may then result in allergic reactions, which in severe cases may have a lethal course. Patients may also develop high titers of antibodies against factor VIIa rendering subsequent treatment difficult or ineffective. Accordingly, a factor VIIa preparation with less tendency to proteolytic degradation in vitro will be more satisfactory and potentially more useful in factor VIIa therapy.

Factor VIIa is probably, like other circulating proteins, removed from the bloodstream by means of enzymatic degradation. In the initial step of this regulatory process the biologically active enzyme is cleaved at one or a few sensitive peptide bonds to produce an inactive degraded molecule. It is very likely that the peptide bonds which are the most sensitive to enzymatic hydrolysis in vivo are identical to the labile peptide bonds which are most frequently observed to be hydrolyzed during production, purification and/or storage of factor VIIa (George J. Broze, Jr., Scot Hichman and Joseph P. Miletich, J.Clin.Invest. 76 (1985) 937-946).

Factor VII contains 17 lysine (positions 18, 32, 38, 62, 85, 109, 137, 143, 148, 157, 161, 197, 199, 316, 337, 341, 389) and 24 arginine (positions 9, 15, 28, 36, 79, 110, 113, 144, 152, 202, 223, 224, 247, 266, 271, 277, 290, 304, 315, 353, 379, 392, 396, 402) residues that in principle all are susceptible to proteolytic degradation, but usually a number of these residues are not active as cleaving sites.

Although the exact halflife of circulating factor VIIa is unknown, preliminary results suggest that factor VIIa procoagulant activity is rapidly cleared from the bloodstream upon intravenous administration (Ulla Hedner and Walter Kisiel, J.Clin.Invest. 71 (1983) 1836-1841).

The treatment and the lives of the patients will be negatively influenced by the observed short in vivo half life of native factor VIIa. Relatively high doses and frequent administration will be necessary to reach and sustain the desired therapeutic or prophylactic effect. As a consequence adequate dose regulation will be difficult to obtain and the need for frequent intravenous administrations will impose restrictions on the patients' way of living.

Consequently, there exists a need in the art for factor VIIa preparations which are stable during production, purification and storage even at high concentrations, and which furthermore have a longer half life and slower clearance from the blood than the native or recombinant factor VIIa. The present invention fulfills this need by providing certain modified factor VII/VIIa.

## DESCRIPTION OF THE INVENTION

In its broadest aspect the present invention provides a modified factor VII/VIIa being stabilized against proteolytic cleavage at certain positions in the molecule. More specifically the present invention provides modified factor VII/VIIa in which one or more proteolytically sensible peptide bond(s) in native factor VII/VIIa has/have been replaced by a proteolytically more stable peptide bond.

According to the present invention this is achieved by modifications at certain positions in the native human factor VII/VIIa molecule. Such modifications may include removal of certain amino acid residues or replacement of one or more amino acid residues with a different amino acid residue. For instance a trypsin like proteolytic cleavage may be hindered by stabilizing the peptide bond on the C-terminal end of certain Arg and/or Lys residues and/or by replacement of certain Arg and/or Lys residues with other amino acid residues and/or by removal of certain Arg and/or Lys residues.

Examples of trypsin-like cleavage sites within the human factor VII molecule at which cleavages have been observed include

(i) lysine(38)-leucine(39),

(ii) lysine(32)-aspartate(33),

(iii) lysine(143)-arginine(144),

(iv) arginine(290)-glycine(291),

(v) arginine(315)-lysine(316),

(vi) lysine(316)-valine(317),

(vii) lysine(341)-glycine(342),

(viii) arginine(392)-serine(393),

(ix) arginine(396)-proline(397) and

(x) arginine(402)-alanine(403).

Minor chymotrypsin-like cleavages have also been observed after

(xi) isoleucine(42) and

(xii) tyrosine(44).

Of these the cleavage sites (i), (ii), (iv) and (v) have been found to be the ones most susceptible to proteolytic degradation, while the remaining are of less quantitative importance.

When considering the stabilization of factor VII/VIIa it is an important aspect that the resulting modified factor VII should retain its activity. This is according to the invention obtained by comparing the sequence of native factor VII/VIIa in the area to be modified with corresponding sequences in related proteins such as factor IX, factor X, factor II and protein C. Homologue sequences around the major cleavage sites are shown below:

```
                                     32      38
                                      |       |
               Factor II       EEAFEALESSTATDVFWAKY
               Factor VII      EEAREIFKDAERTKLFWISY
               Factor X        EEAREVFEDSDKTNEFWNKY
               Factor IX       EEAREVFENTERTTEFWKQY
               Protein C       EEAKEIPONVDDTLAFWSKH


                                          290
                                           |
             Factor II      RVTGWGNLKETWTANVGKGQPSV-L
             Factor VII     LVSGWGQL-------LDRGATALEL
             Factor X       IVSGFGRT-------HEKGRQSTRL
             Factor IX      YVSGWGRV-------FHKGRSALVL
             Protein C      LVTGWGYH-------SSREKEAKRN


                            315                        341
                             |                          |
         Factor II     C-KDSTRI----RITDNMFCAGYKPDEGKRGDACEGDSGGPF
         Factor VII    CLQQSRKVGDSPNITEYMFCAGYS--DGSK-DSCKGDSGGPH
         Factor X      C-----KLSSSFIITQNMFCAGYD--TKQE-DACQGDSGGPH
         Factor IX     CLR-STKFT----IYNNMFCAGFH--EGGR-DSCQGDSGGPH
         Protein C     CSEVMSNM-----VSENMLCAGIL--DGRQ-DACEGDSGGPM
```

Consequently, it is an object of the present invention to provide for modified factor VII/VIIa wherein one, more or all of the lysine, arginine, isoleucine and tyrosine residues:

(i) lysine(38)
(ii) lysine(32)
(iii) lysine(143)
(iv) arginine(290)
(v) arginine(315)
(vi) lysine(316)
(vii) lysine(341)
(viii) arginine(392)
(ix) arginine(396)
(x) arginine(402)
(xi) isoleucine(42) and
(xii) tyrosine(44)

have been stabilized by substitution or deletion.

In a preferred embodiment of the invention one, more or all of the amino acid residues in positions (32), (38), (290) and (315) have been stabilized by substitution or deletion.

According to the present invention Lys in position 32 (ii) and/or 38 (i) may be replaced by another amino acid residue. Lys(38) may preferably be replaced by Thr, Asp, Leu, Gly, Ala, Ser, Asn or His and Lys(32) may preferably be replaced by Gln, Glu, His, Gly, Thr, Ala, or Ser.

Also Arg in position 290 (iv) may be replaced by another amino acid residue, for instance Gly, Ala, Ser, Thr or Lys, preferably Ser, Ala or Gly.

Arg(315) (v) may preferably be substituted by Gly, Thr, Ala, Ser or Gln.

Furthermore Lys(341) (vii) may be substituted by Glu, Gln, Gly, Thr, Ala or Ser, preferably Glu or Gln.

Besides substitution of the above mentioned Arg respective Lys residues with another amino acid residue removal of the Arg or Lys amino acid residues may also be considered in order to avoid proteolytic cleavage. Furthermore, one or more of the amino acid residues on either the N- or C-terminal side of such Arg or Lys residues may be substituted by another amino acid residue exerting a stabilizing effect on the proteolytically sensible peptide bond. An example of such modifications is substitution of the amino acid residue linked to the C-terminal end of a Lys or Arg residue with Pro.

To avoid proteolytic cleavage at position 42 (xi) and 44 (xii), Ile(42) and/or Tyr(44) may be substituted by Asn, Ser, Ala or Gln.

The present invention is contemplated to cover any combination of the above mentioned substitutions and deletions.

Other aspects of the invention will become evident upon reference to the following detailed description and attached drawings.

BRIEF DESCRIPTION OF THE DRAWINGS

Figure 1 illustrates the amino acid sequence given by one letter abbreviations and the tentative structure for factor VII,

Figure 2 illustrates the construction of plasmid pFW10-3/6,

Figure 3 illustrates the construction of plasmid pFW60-3/6, and

Figure 4 illustrates the construction of plasmid pFWx-3/6.

DEFINITIONS

Prior to setting forth the invention, it may be helpful to an understanding thereof to set forth definitions of certain terms to be used hereinafter.

Complementary DNA or cDNA:

A DNA molecule or sequence which has been enzymatically synthesized from the sequences present in a mRNA template or a clone of such molecule.

DNA Construct:

A DNA molecule, or a clone or such a molecule, either single- or double-stranded, which may be isolated in partial form from a naturally occurring gene or which has been modified to contain segments of DNA which are combined and juxtaposed in a manner which would not otherwise exist in nature.

Plasmid or Vector:

A DNA construct containing genetic information which may provide for its replication when inserted into a host cell. A plasmid generally contains at least one gene sequence to be expressed in the host cell; as well as sequences encoding functions which facilitate such gene expression, including promoters and transcription initiation sites. It may be a linear or closed circular molecule. As used herein, the term expression vector shall mean a plasmid or vector containing a transcription promoter and terminator operably linked to a DNA sequence encoding a protein or polypeptide of interest. Expression vectors may further contain other elements, including selectable markers, enhancers, polyadenylation signals, etc., which will be determined in part by the particular host cell chosen.

Biological Activity:

A function or set of functions performed by a molecule in a biological context (i.e. in an organism or an in vitro facsimile). Biological activities of proteins may be divided into catalytic and effector activities. Catalytic activities of clotting factors generally involve the activation of other factors through the specifid cleavage of precursors. Effector activities include specific binding of the biologically active molecule to calcium or other small molecules, to macromolecules such as proteins or to cells. Effector activity frequently augments, or is essential to, catalytic activity under physiological conditions. Catalytic and effector activities may, in some cases, reside within the same domain of a protein.

For factor VIIa biological activity is characterized by the mediation of blood coagulation through the extrinsic pathway. Factor VIIa activates factor X to factor Xa, which in turn converts prothrombin to thrombin thereby initiating the formation of a fibrin clot.

The modified factor VIIa according to the present invention has a biological activity that is substantially the same as that of native factor VIIa.

"Factor VII/VIIa" as used in this application means a product consisting of either the unactivated form (factor VII) or the activated form (factor VIIa) or mixtures thereof. "Modified factor- VII/VIIa" shall mean a biologically active molecule derived from factor VII/VIIa by the substitution or deletion of one or more amino acid residues.

5

As the modifications according to the present invention is made on gene expression level modifications introduced in the factor VII molecule will also be found in the activated product (factor VIIa).

"Factor VII/VIIa" within the above definition includes proteins that have the amino acid sequence of native human factor VII/VIIa. It also includes proteins with a slightly modified amino acid sequence for instance a modified N-terminal end including N-terminal amino acid deletions or additions so long as those proteins substantially retain the activity of factor VIIa.

"Factor VII" within the above definition also includes natural allelic variations that may exist and occur from one individual to another. Also degree and location of glycosylation or other post-translation modifications may vary depending on the chosen host cells and the nature of the host cellular environment.

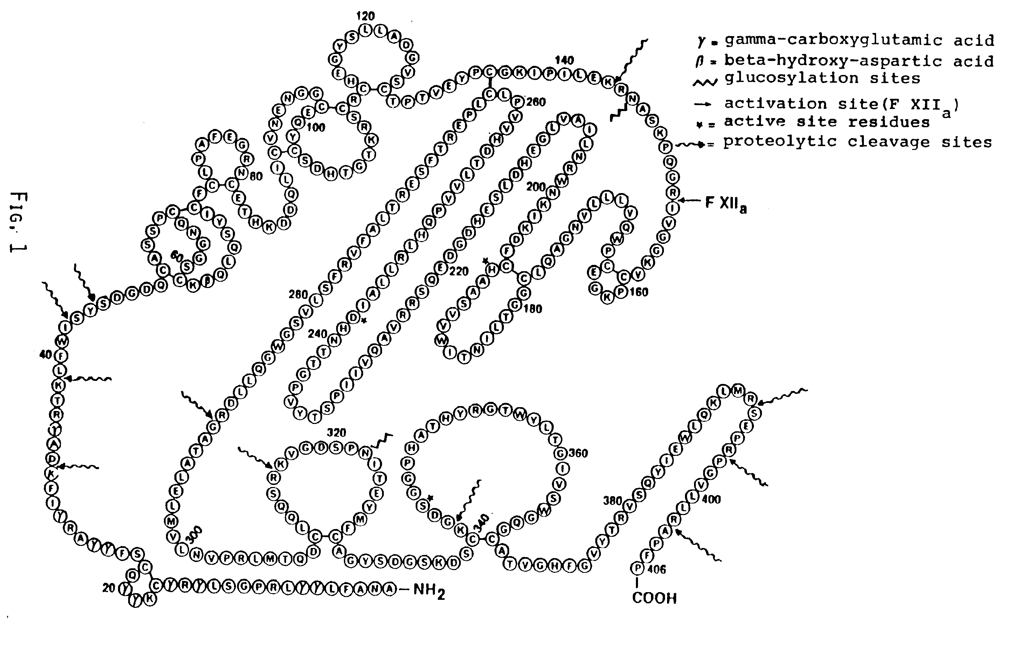

The number system of the amino acid sequence of factor VII/VIIa used herein appears from Figure 1 in which the N-terminal alanine is numbered 1 and the C-terminal proline is numbered 406.

The three letter and one letter abbreviations used for the amino acids are those as normally used in the art, i.e.:

| Amino acid | Three letter abbreviation | One letter abbreviation |
|---|---|---|
| Alanine | Ala | A |
| Cysteine | Cys | C |
| Asparatate | Asp | D |
| Glutamate | Glu | E |
| Phenylalanine | Phe | F |
| Glycine | Gly | G |
| Histidine | His | H |
| Isoleucine | Ile | I |
| Lysine | Lys | K |
| Leucine | Leu | L |
| Methionine | Met | M |
| Asparagine | Asn | N |
| Proline | Pro | P |
| Glutamine | Gln | Q |
| Arginine | Arg | R |
| Serine | Ser | S |
| Threonine | Thr | T |
| Valine | Val | V |
| Tryptophan | Trp | W |
| Tyrosine | Tyr | Y |
| $\gamma$-carboxyglu-tamic acid | Gla | $\gamma$ |

## Best Mode for Carrying out the Invention

The amino acid changes are preferably introduced by oligonucleotide-directed site specific mutagenesis in factor VII cDNA. After screening E. Coli cells the mutated factor VII gene is isolated and recloned into a suitable expression vector. The expression vector is then transfected into an appropriate host cell which when cultured in a suitable culture medium expresses and secretes the modified factor VII which after recovery from

EP 0 370 036 B1

the culture medium is converted into the corresponding modified factor VIIa by known means.

Various host cells may be used including mammalian cells, yeast and other fungi, and bacteria. However, mammalian cells are preferred. A particularly preferred mammalian cell line is the BHK cell line tk⁻tsl3 (Waechter and Basserga, Proc.Natl.Acad.Sci. USA 79: 1106-1110, 1982). Methods for expressing cloned genes in each of these hosts are known in the art, vide for instance EP published patent application No. 200,421 (expression of factor VII and IX in mammalian cells), EP published patent application No. 191,606 (expression of protein C in bacterial cells and EP published patent application No. 167,420 (expression of factor IX in yeast).

For expression of modified factor VII according to the invention in cultured mammalian cells, expression vectors containing cloned modified factor VII sequences are introduced into the cells by appropriate transfection techniques, such as calcium phosphate-mediated transfection (Graham and Van der Eb, Virology 52: 456-467, 1973; as modified by Wigler et al., Proc.Natl.Acad.Sci. USA 77: 3567-3570, 1980). Electroporation transfection technique may also be used (Neuman et al., EMBO.J. I: 841-845, 1982). A DNA-calcium phosphate precipitate is formed, and this precipitate is applied to the cells. A portion of the cells take up the DNA and maintain it inside the cell for several days. A small fraction of the cells integrate the DNA into the genome of the host cell. These integrants are identified by cotransfection with a gene that confers a selectable phenotype (a selectable marker). A preferred selectable marker is the mouse dihydrofolate reductase (DHFR) gene, which imparts cellular resistance to the drug methotrexate (MTX). After the host cells have taken up the DNA, drug selection is applied to select for a population of cells that are expressing the selectable marker in a stable fashion.

Modified factor VII produced by the transfected cells may be removed from the cell culture media by adsorption to barium citrate. Spent medium is mixed with sodium citrate and barium chloride and the precipitate collected. The precipitated material may then be assayed for the presence of the appropriate clotting factor. Further purification may be achieved through immunoadsorption. It is preferred that the immunoadsorption column comprise a high-specificity monoclonal antibody. Alternatively, purification of the barium citrate precipitated material may be accomplished by more conventional biochemical methods or by high-performance liquid chromatography (HPLC).

Conversion of single-chain modified factor VII to active two-chain modified factor VIIa may be achieved using factor XIIa as described by Hedner and Kisiel (J.Clin.Invest. 71: 1836-1841, 1983), or with other proteases having trypsin-like specificity (Kisiel and Fujikawa, Behring Inst. Mitt. 73: 29-42, 1983). Alternatively modified factor VII may be activated by passing it through an ion-exchange chromatography column, such as mono Q® (Pharmacia Fire Chemicals) or the like (Bjoern et al., Research Disclosures, 269, September 1986, pp. 564 - 565).

The following examples are offered by way of illustration and not by way of limitation.

EXAMPLES

MATERIALS AND METHODS

Restriction enzymes were obtained from Bethesda Research Laboratories (BRL), New England Biolabs, and Stratagene and were used as indicated by the producer, unless otherwise stated, Oligonucleotides were synthesized on an automatic DNA synthesizer using phosphoramidite chemistry on a controlled pore glass support (S.L. Beaucage and M.H. Caruthers (1981) Tetrahydron Letters 22, 1859 - 1869). E. coli cells were transformed as described by Maniatis et al. (Molecular Cloning: A Laboratory Manual, Cold Spring Harbour Laboratory, 1982).

A representative modified factor VII was prepared by changing amino acid No. 32 from Lys - → Gln and amino acid No. 38 from Lys - → Thr by oligonucleotide directed mutagenesis.

The oligonucleotides with these changes are of the sequences:

I)                                    BglII          Lys(32)

cDNA:                      ... ... GAG ... ... AAG ... ... ...
                                        ↓               ↓
oligonucleotide: 5' GCC CGG GAA ATC TTC CAG GAC GCG GAG 3'
                                        ↑               ↑
       nucleotide position     228             235
                               Glu             Gln

which is a 27-mer with changes at nucleotide position 228 which destroy a BglII site without changing the amino acid and at position 235 changing amino acid Lys(32) to Gln.

II)                                    Lys(38)

cDNA                       ... ... AAG ... ... ... ...
                                        ↓
oligonucleotide: 5' AGG ACG ACG CTG TTC TGG ATT 3'
                                        ↑
       nucleotide position     254
                               Thr

which is a 21-mer with changes at nucleotide position 254 changing amino acid Lys(38) to Thr.

A further representative modified factor VII was prepared by changing amino acid No. 290 from Arg -- Ser and amino acid No. 315 from Arg -- Ser by oligonucleotide directed mutagenesis.

The oligonucleotides with these changes are of the sequences:

(III)                                  Arg

cDNA:                      ...  ... ... CGT ... ... ... ... ..
                                        ↓
oligonucleotide: 5' GCTG CTG GAC AGT GGC GCC ACG GCC CT
                                        ↑
       nucleotide position     1009

                               Ser

which is a 27-mer with changes at nucleotide position 1009 changing the amino acid Arg (290) to Ser.

```
(IV)                                        Arg

cDNA                    .... ... ... CGG ... ... ... ... .
                                      ↓ ↓
oligonucleotide: 5' GCAG CAG TCA AGT AAG GTG GGA GAC T
                                      ↑ ↑
    nucleotide position:      1084 1086

                                        Ser
```

which is a 26-mer with changes at nucleotide position 1084 and 1086 changing amino acid Arg(315) to Ser.

Example 1

Production of a modified factor VIIa in which Lys(32) has been replaced with Gln (factor VIIa(Gln(32))).

**Recloning Factor VII cDNA**

Factor VII cDNA with a 38 amino acid long leader (Berkner, K.L. et al., Cold Spring Harbor Symposium on Quantitative Biology, Vol. LI, 531-541, 1986) was cloned in the EcoRI site of pGEM3 vector (Promega Biotec) and propagated in E. coli MC 1061 (dam⁺) or MC 1000 (dam⁻) bacteria strain.

Briefly, plasmid FVII(565 + 2463)/pDK was cut with EcoRI and the factor VII cDNA was ligated to EcoRI cut pGEM3. The construction of plasmid FVII(565 + 2463)/pDX is described in EP patent application No. 86302855.1. The plasmid has also been deposited at American Type Culture Collection (ATTC No. 40205).

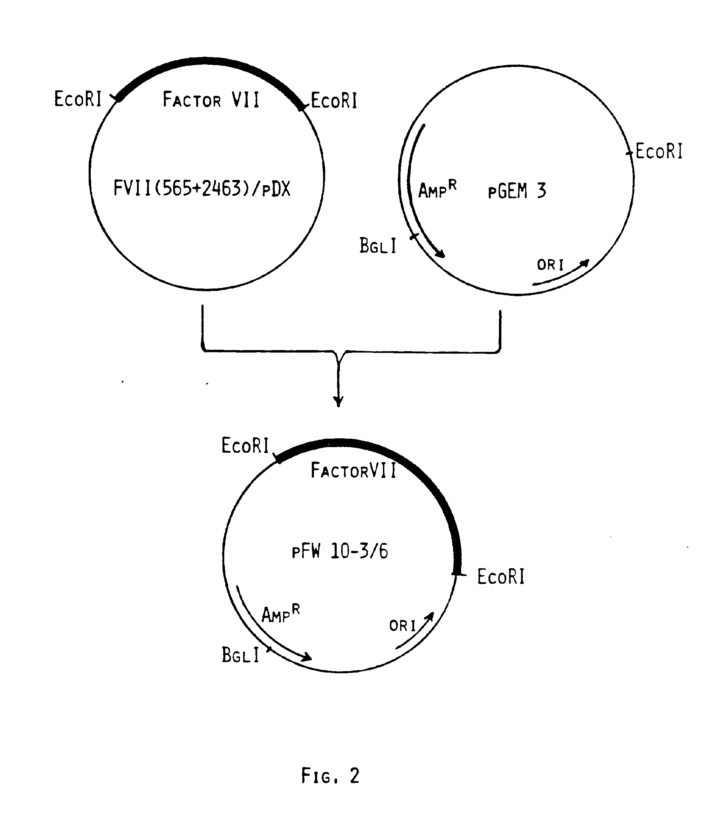

Small and large scale DNA preparations were prepared as described in for example Maniatis et al., Molecular Cloning, A Laboratory Manual, Cold Spring Harbour 1982. One of these plasmid preparations is termed pFW 10-3/6. The construction of pFW 10-3/6 is illustrated in fig. 2.

**Addition of 5′ phosphate groups to oligonucleotides**

Addition of either labelled or unlabelled phosphate groups to oligonucleotides was carried out as described (Maniatis et al. as above).

**Oligonucleotide directed site specific mutagenesis using double-stranded plasmid DNA**

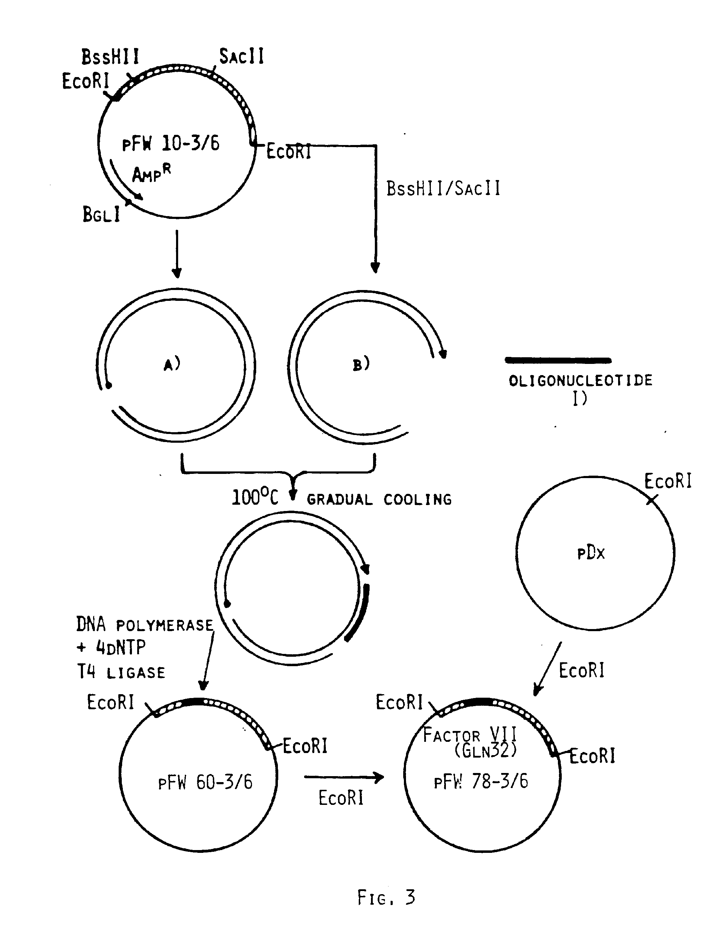

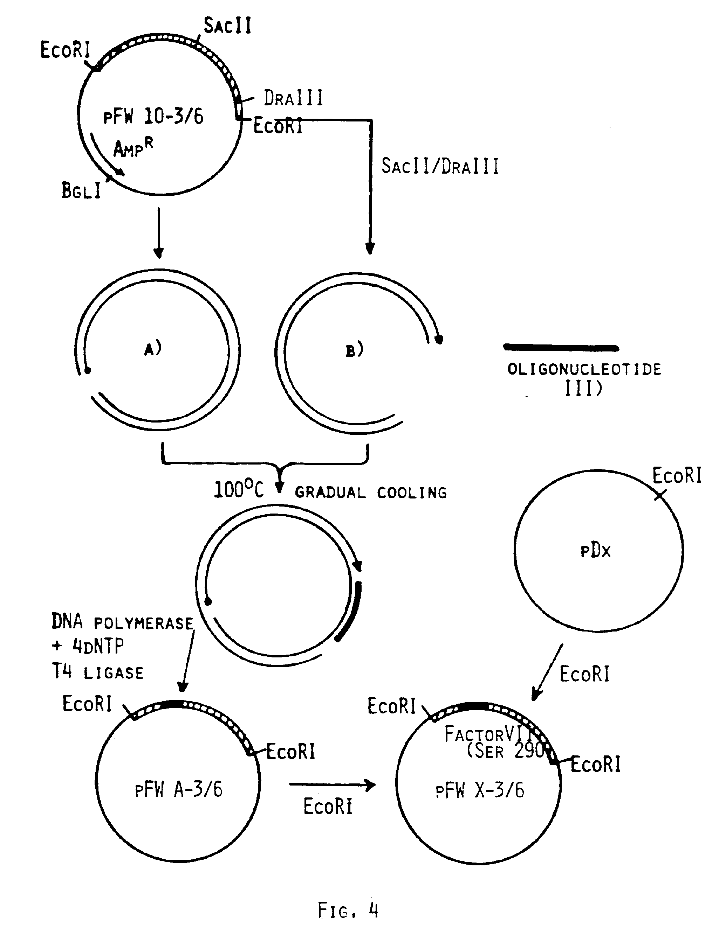

The site directed mutagenesis reaction was carried out by modifying the method by Morinaga et al. 1984 (BIO/TECHNOLOGY vol. 2 p. 636). Plasmid pFW10-3/6 containing FVII cDNA was digested with BglI, a unique site in the plasmid. This cleavage generated fragment a) shown in fig. 3 and fig. 4 destroying the ampicillin resistance. Fragment a) was purified by electroelution from agarose gel and treated with calf intestinal alkaline phosphatase (CIAP) as described in Maniatis et al. as above.

Another sample of pFW 10-3/6 was digested with BssHII and SacII generating fragment b) in fig. 3 with a window of 575 bp in the FVII cDNA. Fragment b) was purified by electroelution from agarose gel after electrophoresis.

Fragments a) and b) were further purified by several phenol extractions, phenol/chloroform (1:1, v/v) and chloroform/isoamyl alcohol (24:1, v/v) extractions, precipitated with 0.3 M Na-acetate and 70% (v/v) ethanol and dissolved in TE (10 mM Tris, 1 mM EDTA pH 7.6).

Then 0.1 - 0.15 pmol of both fragment a) and b) were mixed with 25 pmol of the phosphorylated synthetic oligonucleotide I) in an Eppendorf tube.

Then 10 µl of 5Xpolymerase-ligase buffer (0.5 M NaCl, 33 mM Tris HCl pH 7.5, 40 mM MgCl₂, 5 mM 2-ME) were added.

From the final mixture 15 µl samples were removed and stored on ice until later use as marker in gel electrophoresis. The remaining mixture was incubated in a boiling water bath for 4 min. to denature the DNA fragments. After incubation the mixture was gradually cooled. Upon reannealing heteroduplexes were formed and using agarose gel electrophoresis the formation of a new circular DNA with the correct mutation was demonstrated by comparison with the non-heated sample from above.

Then 10 µl of the four deoxyribonucleoside triphosphate (2.5 mM each), 3 µl of 20 mM ATP, 1 µl of Klenow fragment of DNA polymerase I (5 U/µl) and 1 µl of T4 DNA ligase (10 U/µl) were added to the mixture (20 µl) of heteroduplexes (final volume 40 µl). The final mixture was incubated at 12°C overnight.

Transformation of E. coli MC 1061 and MC 1000 with the incubation mixture resulted in ampicillin resistant transformants. Transformants carrying the mutant FVII gene were selected by colony hybridization (Maniatis et al.) with the 5'-$^{32}$P-labelled 27-mer and 21-mer synthetic oligonucleotides.

After retransformation plasmid DNA was purified from selected colonies, analysed, and sequenced (by the Maxam-Gilbert method and the dideoxy method) to verify the mutation caused by the synthetic oligonucleotide.

The construction of plasmid pFW 60-3/6 harbouring a mutated factor VII gene in which Lys(32) has been replaced with Gln is illustrated in fig. 3.

pFW 60-3/6 was digested with EcoRI and the EcoRI-EcoRI factor VII fragment was ligated into EcoRI cut pDx plasmid to obtain plasmid pFW 78-3/6 harbouring the factor VII(Gln32) gene in the same orientation as in plasmid FVII(565 + 2463)/pDX. Plasmid pFW 78-3/6 was then transfected into BHKtk⁻tsl3 cells following the general procedure described above.

The modified factor VII produced by the cells is then precipitated with barium citrate; purified by immunoadsorption; and activated to modified factor VIIa by passing it through an ion-exchange chromatography column as described by Bjoern et al., supra.

Test for Activity

As activated native factor VIIa, the activated modified factor VIIa shortened the coagulation period in a one-stage clotting assay. The activated modified factor VIIa was incubated at a concentration of approximately 0,9 mg/ml in a 10 mM Tris-HCl buffer at pH 8.5 comprising 390 mM NaCl and 5 mM EDTA. The degradation was monitored by SDS-PAGE of reduced samples and when significant degradation had occured an aliqot was withdrawn and applied to an HPLC column. The preparative chromatography served mainly to exclude Tris from the sample for amino acid sequencing as intact and degraded modified factor VIIa coeluted from the column. N-terminal amino acid sequencing revealed that no hydrolysis had occurred of the peptide bond between glutamine residue No. 32 and aspartic acid residue No. 33. In contrast profound degradation at lysine residue No. 32 was observed when activated native factor VIIa was subjected to the same treatment and analysis as performed in a parallel investigation.

Example 2

Production of a modified factor VIIa in which Lys(38) has been replaced with Thr (factor VIIa(Thr 38)).

By following the procedure of example 1 with the only exception that the synthetic oligonucleotide II) was used instead of I) an expression plasmid was obtained harbouring the mutated factor VII gene.

This plasmid is then transfected into BHKtk⁻tsl3 cells and factor VII(Thr38) is recovered from the cell supernatant and activated to factor VIIa(Thr38) as described.

Example 3

Production of a modified factor VIIa in which Lys(32) and Lys(38) have been replaced with Gln and Thr, respectively (factor VIIa(Gln32, Thr38)).

By following the procedure of example 1 with the only exception that 12.5 pmol of both oligonucleotide I) and II) are used in the site directed mutagenesis reaction, an expression plasmid harbouring the mutated factor VII gene was obtained.

This plasmid is then transfected into BHKtk⁻tsl3 cells and factor VII(Gln32, Thr38) is recovered from the cell supernatant and activated to factor VIIa(Gln32, Thr38) as described.

Example 4

Production of a modified factor VIIa in which Arg(290) has been replaced with Ser (factor VIIa(Ser(290))).

The construction of plasmid pFW A-3/6 harbouring a mutated factor VII gene in which Arg(290) has been replaced with Ser is illustrated in fig. 4.

Plasmid pFW 10-3/6 was used to produce the fragment a) of Example 1; and another sample of pFW 10-3/6 was digested with SacII and DraIII generating fragment b) in fig. 4 with a window of 1366 bp in the FVII cDNA.

Fragments b) and a) were subsequently treated as described in Example 1 except for the use of oligonucleotide III) instead of I).

pFW A-3/6 was digested with EcoRI and the EcoRI-EcoRI factor VII fragment was ligated into EcoRI cut pDx plasmid to obtain plasmid pFW X-3/6 harbouring the factor VII(Ser290) gene in the same orientation as in plasmid FVII(565 + 2463)/pDX. Plasmid pFW X-3/6 was then transfected into BHKtk⁻tsl3 cells following the general procedure described above.

The modified factor VII produced by the cells is then precipitated with barium citrate; purified by immunoadsorption; and activated to modified factor VIIa by passing it through an ion-exchange chromatography column as described by Bjoern et al., supra.

Example 5

Production of a modified factor VIIa in which Arg(315) has been replaced with Srr (factor VIIa(Ser315)).

By following the procedure of example 4 with the only exception that the synthetic oligonucleotide IV) was used instead of III) an expression plasmid was obtained harbouring the mutated factor VII gene.

This plasmid is then transfected into BHKtk⁻tsl3 cells and factor VII(Ser315) is recovered from the cell supernatant and activated to factor VIIa(Ser315) as described

Example 6

Determination of Three Active Proteolytic Cleavage Sites

In order to identify some active cleavage sites a heavily degraded preparation of recombinant factor VII was submitted to N-terminal sequence analysis by automated Edman degradation using an Applied Biosystems model 470 A gas-phase sequencer. The results are shown in Table 1 below

Table 1

| Cycle No. | PTH-a.a. | Yield (pmol) | PTH-a.a. | Yield (pmol) | PTH-a.a. | Yield (pmol) | PTH-a.a. | Yield (pmol) |
|---|---|---|---|---|---|---|---|---|
| 1 | Leu | 593 | Ile | 756 | Gly | 706 | Lys | 425 |
| 2 | Phe | 497 | Val* | 1183 | Ala | 232 | Val* | 1183 |
| 3 | Trp | 341 | Gly* | 1128 | Thr | 38 | Gly* | 1128 |
| 4 | Ile | 458 | Gly | 780 | Ala | 166 | Asp | 413 |
| 5 | Ser* | 213 | Lys | 936 | Leu | 262 | Ser* | 213 |
| 6 | Tyr | 397 | Val | 885 | Glu | 154 | Pro | 386 |
| 7 | Ser | 94 | (1/2Cys) | n.d. | Leu | 240 | Asn** | n.d. |
| 8 | Asp | 312 | Pro | 690 | Met | 136 | Ile | 416 |
| 9 | Gly | 400 | Lys | 815 | Val | 289 | Thr | 104 |
| 10 | Asp | 278 | Gly | 647 | Leu | 225 | Glu | 354 |
| 11 | Gln | 274 | Glu | 670 | Asn | 122 | Tyr | 389 |
| 12 | (1/2Cys) | n.d. | (1/2Cys) | n.d. | Val | 267 | Met | 363 |
| 13 | Ala | 379 | Pro* | 604 | Pro* | 604 | Phe | 370 |
| 14 | Ser | 31 | Trp | 8 | Arg | 270 | (1/2Cys) | n.d. |
| 15 | Ser | 66 | Gln | 394 | | | Ala | 427 |

(*)   The total amount of amino acid residues occuring from two sequences is given

(**) Glycosylated  Asn

n.d. Not determined

In this sample four N-terminals were deduced Leu-39 (column 1), Gly-291 (column 3) and Lys-316 (column 4) corresponding to proteolytic cleavage and Ile-153 (column 2) corresponding to activation of FVII to FVIIa.

**Claims**

1. Modified factor VII/VIIa, characterized in that one, more, or all the lysine, arginine, isoleucine, and tyrosine residues
   (i) Lys(38)
   (ii) Lys(32)
   (iii) Lys(143)
   (iv) Arg(290)
   (v) Arg(315)
   (vi) Lys(316)
   (vii) Lys(341)
   (viii) Arg(398)
   (ix) Arg(396)
   (x) Arg(402)
   (xi) Ile(42)
   (xii) Tyr(44)
have been substituted and/or deleted.

2. Modified factor VII/VIIa according to claim 1, characterized in that one, more or all of the amino acid residues (i), (ii), (iv), and (v) have been substituted or deleted.

3. Modified factor VII/VIIa according to claim 1 or 2, characterized in that Lys(38) has been replaced with Thr, Asp, Leu, Gly, Ala, Ser, Asn, or His.

4. Modified factor VII/VIIa according to claim 3, characterized in that Lys(38) has been replaced with Thr.

5. Modified factor VII/VIIa according to claim 1 or 2, characterized in that Lys(32) has been replaced with Gln, Glu, His, Gly, Thr, Ala, or Ser.

6. Modified factor VII/VIIa according to claim 5, characterized in that Lys(32) has been replaced with Gln.

7. Modified factor VII/VIIa according to claim 1 or 2, characterized in that Arg(290) has been replaced with Gly, Ala, Ser, Thr, or Lys.

8. Modified factor VII/VIIa according to claim 1 or 2, characterized in that Lys(38) has been replaced with Ser, Ala, or Gly.

9. Modified factor VII/VIIa according to claim 1 or 2, characterized in that Arg(315) has been replaced with Gly, Thr, Ser, Ala, or Gln.

10. Modified factor VII/VIIa according to claim 1 or 2, characterized in that Lys(341) has been replaced with Glu, Gln, Thr, Ser, Ala, or Gly, preferably Glu or Gln.

11. Modified factor VII/VIIa according to claim 1, characterized in that Ile(42) has been replaced with Asn, Ser, Ala, or Gln.

12. Modified factor VII/VIIa according to claim 1, characterized in that Tyr(44) has been replaced with Asn, Ser, Ala, or Gln.

13. Modified factor VII/VIIa according to claim 1 or 2, characterized in that it comprises any combination of the substitutions according to any of the claims 3 to 12.

14. Modified factor VII/VIIa according to claim 1 or 2, characterized in that Lys(38) has been replaced with Thr, and Lys(32) has been replaced with Gln.

15. DNA-sequences, characterized in that they encode for a modified factor VII/VIIa according to any of the claims 1 to 14.

16. Expression vectors, characterized in that they contain a DNA-sequence according to claim 15.

17. Cells, characterized in that they are transformed to produce a modified factor VII/VIIa according to any of the claims 1 to 14

18. A method for the production of modified factor VII/VIIa, characterized in that a gene encoding for the expression of factor VII is mutated in one, more or all of the codons responsible for the expression of the lysine, arginine, isoleucine, and tyrosine residues
   (i) Lys(38)
   (ii) Lys(32)
   (iii) Lys(143)
   (iv) Arg(290)
   (v) Arg(315)

13

(vi) Lys(316)
(vii) Lys(341)
(viii) Arg(398)
(ix) Arg(396)
(x) Arg(402)
(xi) Ile(42)
(xii) Tyr(44)

in native factor VII, generating a mutated gene capable of expressing a modified factor VII, wherein one, more or all of said amino acid residues are substituted or deleted, said mutated gene is inserted into a suitable host capable of expressing said mutated gene, said host is cultured in an appropriate growth medium thereby expressing the modified factor VII/VIIa, which subsequently is isolated and optionally activated to generate a modified factor VIIa product.

19. The method of claim 18, characterized in that in the produced modified factor VII/VIIa one, more or all of the amino acid residues (i), (ii), (iv), and (v) have been substituted or deleted.

20. The method of claim 18 or 19, characterized in that in the produced modified factor VII/VIIa Lys(38) has been replaced with Thr, Asp, Leu, Gly, Ala, Ser, Asn, or His.

21. The method of claim 20, characterized in that in the produced modified factor VII/VIIa Lys(38) has been replaced with Thr.

22. The method of claim 18 or 19, characterized in that in the produced modified factor VII/VIIa Lys(32) has been replaced with Gln, Glu, His, Gly, Thr, Ala, or Ser.

23. The method of claim 22, characterized in that in the produced modified factor VII/VIIa Lys(32) has been replaced with Gln.

24. The method of claim 18 or 19, characterized in that in the produced modified factor VII/VIIa Arg(290) has been replaced with Gly, Ala, Ser, Thr, or Lys.

25. The method of claim 18 or 19, characterized in that in the produced modified factor VII/VIIa Lys(38) has been replaced with Ser, Ala, or Gly.

26. The method of claim 18 or 19, characterized in that in the produced modified factor VII/VIIa Arg(315) has been replaced with Gly, Thr, Ser, Ala, or Gln.

27. The method of claim 18, characterized in that in the produced modified factor VII/VIIa Lys(341) has been replaced with Glu, Gln, Thr, Ser, Ala, or Gly, preferably Glu or Gln.

28. The method of claim 18, characterized in that in the produced modified factor VII/VIIa Ile(42) has been replaced with Asn, Ser, Ala, or Gln.

29. The method of claim 18, characterized in that in the produced modified factor VII/VIIa Tyr(44) has been replaced with Asn, Ser, Ala, or Gln.

30. The method of claim 18, characterized in that in the produced modified factor VII/VIIa any combination of the substitutions according to any of the claims 20 to 29 is present.

31. The method of claim 18 or 19, characterized in that in the produced modified factor VII/VIIa Lys(38) has been replaced with Thr, and Lys(32) has been replaced with Gln.

## Revendications

1. Facteur VII/VIIa modifié, caractérisé en ce que un, plusieurs, ou tous les résidus lysine, arginine, isoleucine et tyrosine

(i) Lys(38)
(ii) Lys(32)
(iii) Lys(143)
(iv) Arg(290)
(v) Arg(315)
(vi) Lys(316)
(vii) Lys(341)
(viii) Arg(398)
(ix) Arg(396)
(x) Arg(402)
(xi) Ile(42)
(xii) Tyr(44)

ont été substitués et/ou supprimés par délétion.

2. Facteur VII/VIIa modifié selon la revendication 1, caractérisé en ce que un, plusieurs, ou tous les sidus acides aminés (i), (ii), (iv), et (v) ont été substitués ou supprimés par délétion.

3. Facteur VII/VIIa modifié selon la revendication 1 ou 2, caractérisé en ce que Lys(38) a été remplacé par Thr, Asp, Leu, Gly, Ala, Ser, Asn, ou His.

4. Facteur VII/VIIa modifié selon la revendication 3, caractérisé en ce que Lys(38) a été remplacé par Thr.

5. Facteur VII/VIIa modifié selon la revendication 1 ou 2, caractérisé en ce que Lys(32) a été remplacé par Gln, Glu, His, Gly, Thr, Ala, ou Ser.

6. Facteur VII/VIIa modifié selon la revendication 5, caractérisé en ce que Lys(32) a été remplacé par Gln.

7. Facteur VII/VIIa modifié selon la revendication 1 ou 2, caractérisé en ce que Arg(290) a été remplacé par Gly, Ala, Ser, Thr, ou Lys.

8. Facteur VII/VIIa modifié selon la revendication 1 ou 2, caractérisé en ce que Lys(38) a été remplacé par Ser, Ala, ou Gly.

9. Facteur VII/VIIa modifié selon la revendication 1 ou 2, caractérisé en ce que Arg(315) a été remplacé par Gly, Thr, Ser, Ala, ou Gln.

10. Facteur VII/VIIa modifié selon la revendication 1 ou 2, caractérisé en ce que Lys(341) a été remplacé par Glu, Gln, Thr, Ser, Ala, ou Gly, de préférence par Glu ou Gln.

11. Facteur VII/VIIa modifié selon la revendication 1, caractérisé en ce que Ile(42) a été remplacé par Asn, Ser, Ala, ou Gln.

12. Facteur VII/VIIa modifié selon la revendication 1, caractérisé en ce que Tyr(44) a été remplacé par Asn, Ser, Ala, ou Gln.

13. Facteur VII/VIIa modifié selon la revendication 1 ou 2, caractérisé en ce qu'il comprend une combinaison quelconque des substitutions selon l'une quelconque des revendications 3 à 12.

14. Facteur VII/VIIa modifié selon la revendication 1 ou 2, caractérisé en ce que Lys(38) a été remplacé par Thr, et Lys(32) a été remplacé par Gln.

15. Séquences de DNA, caractérisées en ce qu'elles codent pour un facteur VII/VIIa modifié selon l'une quelconque des revendications 1 à 14.

16. Vecteur d'expression, caractérisés en ce qu'ils contiennent une séquence de DNA selon la revendication 15.

17. Cellules, caractérisées en ce qu'elles sont transformées pour produire un facteur VII/VIIa modifié selon l'une quelconque des revendications 1 à 14.

18. Procédé pour la production d'un facteur VII/VIIa modifié, caractérisé en ce qu'un gène codant pour l'expression du facteur VII est muté en un, plusieurs ou tous les codons responsables de l'expression des résidus lysine, arginine, isoleucine, et tyrosine.

(i) Lys(38)
(ii) Lys(32)
(iii) Lys(143)
(iv) Arg(290)
(v) Arg(315)
(vi) Lys(316)
(vii) Lys(341)
(viii) Arg(398)
(ix) Arg(396)
(x) Arg(402)
(xi) Ile(42)
(xii) Tyr(44)

dans le facteur VII natif, générant un gène muté capable d'exprimer un facteur VII modifié, dans lequel un, plusieurs ou tous les résidus acides aminés sont substitués ou supprimés par délétion, ledit gène muté est inséré dans un hôte convenable capable d'exprimer ce gène muté, ledit hôte est cultivé dans un milieu de croissance approprié exprimant de ce fait le facteur VII/VIIa modifié, qui par la suite est isolé et facultativement activé pour générer un produit du facteur VIIa modifié.

19. Procédé selon la revendication 18, caractérisé en ce que dans le facteur VII/VIIa modifié produit, un, plusieurs ou tous les résidus acides aminés (i), (ii), (iv) et (v) ont été substitués ou supprimés par délétion.

20. Procédé selon la revendication 18 ou 19, caractérisé en ce que dans le facteur VII/VIIa modifié produit, Lys(38) a été remplacé par Thr, Asp, Leu, Gly, Ala, Ser, Asn, ou His.

21. Procédé selon la revendication 20, caractérisé en ce que dans le facteur VII/VIIa modifié produit, Lys(38) a été remplacé par Thr.

22. Procédé selon la revendication 18 ou 19, caractérisé en ce que dans le facteur VII/VIIa modifié produit, Lys(32) a été remplacé par Gln, Glu, His, Gly, Thr, Ala, ou Ser.

23. Procédé selon la revendication 22, caractérisé en ce que dans le facteur VII/VIIa modifié produit, Lys(32) a été remplacé par Gln.

24. Procédé selon la revendication 18 ou 19, caractérisé en ce que dans le facteur VII/VIIa modifié produit, Arg(290) a été remplacé par Gly, Ala, Ser, Thr, ou Lys.

25. Procédé selon la revendication 189 ou 19, caractérisé en ce que dans le facteur VII/VIIa modifié produit, Lys(38) a été remplacé par Ser, Ala, ou Gly.

26. Procédé selon la revendication 18 ou 19, caractérisé en ce que dans le facteur VII/VIIa modifié produit, Arg(315) a été remplacé par Gly, Thr, Ser, Ala, ou Gln.

27. Procédé selon la revendication 18, caractérisé en ce que dans le facteur VII/VIIa modifié produit, Lys(341) a été remplacé par Glu, Gln, Thr, Ser, Ala, ou Gly, de préférence par Glu, ou Gln.

28. Procédé selon la revendication 18, caractérisé en ce que dans le facteur VII/VIIa modifié produit, Ile(42) a été remplacé par Asn, Ser, Ala, ou Gln.

29. Procédé selon la revendication 18, caractérisé en ce que dans le facteur VII/VIIa modifié produit, Tyr(44) a été remplacé par Asn, Ser, Ala, ou Gln.

30. Procédé selon la revendication 18, caractérisé en ce que dans le facteur VII/VIIa modifié produit, une quelconque combinaison des substitutions selon l'une quelconque des revendications 20 à 29 est présente.

31. Procédé selon la revendication 18 ou 19, caractérisé en ce que dans le facteur VII/VIIa modifié produit, Lys(38) a été remplacé par Thr, et Lys(32) a été remplacé par Gln.


## Patentansprüche

1. Modifizierter Faktor VII/VIIa, dadurch gekennzeichnet, daß einer, mehrere oder alle der Lysin-, Arginin-, Isoleucin- und Tyrosin-Reste

(i) Lys(38)

(ii) Lys(32)

(iii) Lys(143)

(iv) Arg(290)

(v) Arg(315)

(vi) Lys(316)

(vii) Lys(341)

(viii) Arg(398)

(ix) Arg(396)

(x) Arg(402)

(xi) Ile(42)

(xii) Tyr(44)

substituiert und/oder entfernt worden sind.

2. Modifizierter Faktor VII/VIIa nach Anspruch 1, dadurch gekennzeichnet, daß einer, mehrere oder alle der Aminosäure-Reste (i), (ii), (iv) und (v) substituiert oder entfernt worden sind.

3. Modifizierter Faktor VII/VIIa nach Anspruch 1 oder 2, dadurch gekennzeichnet, daß Lys(38) durch Thr, Asp, Leu, Gly, Ala, Ser, Asn oder His ersetzt worden ist.

4. Modifizierter Faktor VII/VIIa nach Anspruch 3, dadurch gekennzeichnet, daß Lys(38) durch Thr ersetzt worden ist.

5. Modifizierter Faktor VII/VIIa nach Anspruch 1 oder 2, dadurch gekennzeichnet, daß Lys(32) durch Gln, Glu, His, Gly, Thr, Ala oder Ser ersetzt worden ist.

6. Modifizierter Faktor VII/VIIa nach Anspruch 5, dadurch gekennzeichnet, daß Lys(32) durch Gln ersetzt worden ist

7. Modifizierter Faktor VII/VIIa nach Anspruch 1 oder 2, dadurch gekennzeichnet, daß Arg(290) durch Gly, Ala, Ser, Thr oder Lys ersetzt worden ist.

8. Modifizierter Faktor VII/VIIa nach Anspruch 1 oder 2, dadurch gekennzeichnet, daß Lys(38) durch Ser, Ala oder Gly ersetzt worden ist

9. Modifizierter Faktor VII/VIIa nach Anspruch 1 oder 2, dadurch gekennzeichnet, daß Arg(315) durch Gly, Thr, Ser, Ala oder Gln ersetzt worden ist.

10. Modifizierter Faktor VII/VIIa nach Anspruch 1 oder 2, dadurch gekennzeichnet, daß Lys(341) durch Glu, Gln, Thr, Ser, Ala oder Gly, bevorzugt durch Glu oder Gln, ersetzt worden ist.

11. Modifizierter Faktor VII/VIIa nach Anspruch 1, dadurch gekennzeichnet, daß Ile(42) durch Asn, Ser, Ala oder Gln ersetzt worden ist.

12. Modifizierter Faktor VII/VIIa nach Anspruch 1, dadurch gekennzeichnet, daß Tyr(44) durch Asn, Ser, Ala oder Gln ersetzt worden ist.

13. Modifizierter Faktor VII/VIIa nach Anspruch 1 oder 2, dadurch gekennzeichnet, daß er irgendeine Kombination der Substitutionen nach einem der Ansprüche 3 bis 12 aufweist.

14. Modifizierter Faktor VII/VIIa nach Anspruch 1 oder 2, dadurch gekennzeichnet, daß Lys(38) durch Thr ersetzt worden ist und Lys(32) durch Gln ersetzt worden ist.

15. DNA-Sequenzen, dadurch gekennzeichnet, daß sie für einen modifizierten Faktor VII/VIIa nach einem der Ansprüche 1 bis 14 codieren.

16. Expressionsvektoren, dadurch gekennzeichnet, dadurch gekennzeichnet, daß sie eine DNA-Sequenz nach Anspruch 15 enthalten.

17. Zellen, dadurch gekennzeichnet, daß sie zum Erzeugen eines modifizierten Faktors VII/VIIa nach einem der Ansprüche 1 bis 14 transformiert werden.

18. Verfahren Für die Herstellung von modifiziertem Faktor VII/VIIa, dadurch gekennzeichnet, daß ein für die Expression des Faktors VII codierendes Gen in eines, mehrere oder alle der Codone mutiert wird, die für die Expression der Lysin-, Arginin-, Isoleucin- und Tyrosin-Reste

(i) Lys(38)

(ii) Lys(32)

(iii) Lys(143)

(iv) Arg(290)

(v) Arg(315)

(vi) Lys(316)

(vii) Lys(341)

(viii) Arg(398)

(ix) Arg(396)

(x) Arg(402)

(xi) Ile(42)

(xii) Tyr(44)

im nativen Faktor VII verantwortlich sind, der ein mutiertes Gen erzeugt, welches zum Exprimieren eines modifizierten Faktors VII fähig ist, in dem einer, mehrere oder alle der Aminosäure-Reste substituiert oder entfernt worden sind, wobei das mutierte Gen in einen zweckmäßigen Wirt eingefügt wird, der fähig ist, das mutierte Gen zu exprimieren, wobei der Wirt in einem geeigneten Wachstumsmedium kultiviert wird, so daß der modifizierte Faktor VII/VIIa exprimiert wird, welcher anschließend isoliert und wahlweise aktiviert wird, um ein Modifizierter-Faktor-VIIa-Produkt zu erzeugen.

19. Verfahren nach Anspruch 18, dadurch gekennzeichnet, daß in dem erzeugten modifizierten Faktor VII-/VIIa einer, mehrere oder alle der Aminosäure-Reste (i), (ii), (iv) und (v) substituiert oder entfernt worden sind.

20. Verfahren nach Anspruch 18 oder 19, dadurch gekennzeichnet, daß in dem erzeugten modifizierten Faktor VII/VIIa Lys(38) durch Thr, Asp, Leu, Gly, Ala, Ser, Asn oder His ersetzt worden ist.

21. Verfahren nach Anspruch 20, dadurch gekennzeichnet, daß in dem erzeugten modifizierten Faktor VII-/VIIa Lys(38) durch Thr ersetzt worden ist.

22. Verfahren nach Anspruch 18 oder 19, dadurch gekennzeichnet, daß in dem erzeugten modifizierten Faktor VII/VIIa Lys(32) durch Gln, Glu, His, Gly, Thr, Ala oder Ser ersetzt worden ist.

23. Verfahren nach Anspruch 22, dadurch gekennzeichnet, daß in dem erzeugten modifizierten Faktor VII-/VIIa Lys(32) durch Gln ersetzt worden ist.

24. Verfahren nach Anspruch 18 oder 19, dadurch gekennzeichnet, daß in dem erzeugten modifizierten Faktor VII/VIIa Arg(290) durch Gly, Ala, Ser, Thr oder Lys ersetzt worden ist.

25. Verfahren nach Anspruch 18 oder 19, dadurch gekennzeichnet, daß in dem erzeugten modifizierten Faktor VII/VIIa Lys(38) durch Ser, Ala oder Gly ersetzt worden ist.

26. Verfahren nach Anspruch 18 oder 19, dadurch gekennzeichnet, daß in dem erzeugten modifizierten Faktor VII/VIIa Arg(315) durch Gly, Thr, Ser, Ala oder Gln ersetzt worden ist.

27. Verfahren nach Anspruch 18, dadurch gekennzeichnet, daß in dem erzeugten modifizierten Faktor VII-/VIIa Lys(341) durch Glu, Gln, Thr, Ser, Ala oder Gly, bevorzugt durch Glu oder Gln, ersetzt worden ist.

28. Verfahren nach Anspruch 18, dadurch gekennzeichnet, daß in dem erzeugten modifizierten Faktor VII-/VIIa Ile(42) durch Asn, Ser, Ala oder Gln ersetzt worden ist.

29. Verfahren nach Anspruch 18, dadurch gekennzeichnet, daß in dem erzeugten modifizierten Faktor VII-/VIIa Tyr(44) durch Asn, Ser, Ala oder Gln ersetzt worden ist.

30. Verfahren nach Anspruch 18, dadurch gekennzeichnet, daß in dem erzeugten modifizierten Faktor VII-/VIIa irgendeine Kombination der Substitutionen nach einem der Ansprüche 20 bis 29 vorliegt.

31. Verfahren nach Anspruch 18 oder 19, dadurch gekennzeichnet, daß in dem erzeugten modifizierten Faktor VII/VIIa Lys(38) durch Thr ersetzt worden ist und Lys(32) durch Gln ersetzt worden ist.

Fig. 1

γ = gamma-carboxyglutamic acid
β = beta-hydroxy-aspartic acid
$\sim\!\sim$ glucosylation sites
→ = activation site (F XII$_a$)
⚹ = active site residues
$\sim\!\!\!\!\to$ = proteolytic cleavage sites

EP 0 370 036 B1

Fig. 2

FIG. 3

FIG. 4